Europäisches Patentamt

European Patent Office

Office européen des brevets

(11) Numéro de publication: **0 011 030**
A1

(12) **DEMANDE DE BREVET EUROPEEN**

(21) Numéro de dépôt: 79400798.9

(22) Date de dépôt: 26.10.79

(51) Int. Cl.³: **G 01 N 33/54**

(30) Priorité. 31.10.78 FR 7831279

(71) Demandeur: Maes, Roland, 10a, rue du 22 Novembre, F-67000 - Strasbourg (FR)

(43) Date de publication de la demande: 14.05.80
Bulletin 80/10

(72) Inventeur: Maes, Roland, 10a, rue du 22 Novembre, F-67000 - Strasbourg (FR)

(84) Etats contractants désignés: AT BE CH DE GB IT LU NL SE

(74) Mandataire: Poupon, Michel, Cabinet Arbousse Bastide 20, rue de Copenhague, F-67000 Strasbourg (FR)

(54) Perfectionnements aux procédés de détermination de substances montrant entr'elles des affinités de liaison spécifiques par des tests faisant intervenir une phase solide.

(57) Procédé pour la détermination de substances montrant entr'elles des affinités de liaison spécifiques, dans lequel on soumet à une réaction de liaison spécifique la substance à déterminer avec un partenaire de liaison primaire en quantité connue et limitée, une partie dudit partenaire de liaison primaire subsistant après achèvement de la réaction de liaison spécifique.

Ledit partenaire de liaison subsistant est adsorbé spécifiquement sur une phase solide consistant en un gel de polyacrylamide sensibilisé par une substance ayant des propriétés liantes spécifiques pour le partenaire de liaison primaire puis détecté par voie enzymatique ou par fluorescence.

EP 0 011 030 A1

La présente invention concerne un perfectionnement aux procédés pour la détermination de substances montrant entr'elles des affinités de liaison spécifiques connues, comme par exemple des haptènes et des antigènes, et leurs anticorps respectifs, procédés du type dans lesquels est utilisée une phase solide sensibilisée par un des éléments qui interviennent dans la réaction spécifique d'affinité entre les deux substances.

On connaît déjà des procédés exploitant l'affinité spécifique de liaison pouvant exister entre deux substances. Ces procédés sont basés sur le principe suivant : une réaction d'affinité liante spécifique est engendrée entre une substance et son partenaire de liaison primaire, attaché à une phase solide constitué d'un gel d'agarose, de grains de cellulose, de latex, ou encore d'une surface de polystyrène (tube ou film). Après que la réaction ait eu lieu, la phase solide est isolée du milieu réactionnel et la quantité de substance à déterminer qui y est spécifiquement liée par une réaction d'affinité liante spécifique est mesurée, soit par l'intermédiaire d'un enzyme attaché au préalable par une liaison covalente à l'un des constituants du système, soit par l'intermédiaire d'un troisième réactif couplé de façon covalente à un enzyme, réactif qui est à son tour spécifiquement liant pour l'un des constituants du système de détection. Le marqueur utilisé est généralement un enzyme mais peut être tout autre élément de détection approprié, par exemple un isotope radioactif, tel l'iode 125 ou le tritium.

La sensibilité de systèmes de détection de substances présentes dans un liquide en quantité inconnue basés sur l'attachement de cette substance de façon spécifique à un partenaire de liaison fixé sur une phase solide, est en général médiocre, spécialement lorsque le marqueur utilisé est un enzyme. Cette sensibilité réduite est principalement due au fait que les réactifs utilisés ne sont pas physico-chimiquement inertes. Ils réagissent entr'eux de façon non-spécifique et ces réactions annexes augmentent considérablement le bruit de fond de la réaction spécifique de liaison étudiée.

En outre, la phase solide adsorbe de façon variable et non-spécifique la majorité des substances avec lesquelles elle entre en contact. C'est ainsi qu'elle adsorbe entre autres les gamma-globulines qui sont très fréquemment utilisées dans ces tests de détection, ceci est encore accentué par la présence nécessaire d'agents pontants comme par exemple la glutaraldéhyde.

D'autre part, les phases solides constituées de polymères de carbohydrates, tels que la cellulose, le sephadex, l'agarose et utilisées communément dans des tests de détection sur phase solide, après activation par des halogénures de cyanogène ou une diazotation, et sensibilisation subséquente par une substance possédant des affinités de liaison spécifiques, subit au cours du temps un phénomène de relargage. Des travaux ont montré qu'une partie de la substance qui leur est attachée de façon covalente se sépare de la matrice solide et celle-ci perd graduellement l'élément sensibilisant, avec perte conséquente de sensibilité.

Enfin, le chromophore produit par la réaction enzymatique finale utilisée dans des tests où le marqueur est un enzyme, a tendance à précipiter sur la phase solide activée. Une détermination d'activité enzymatique réalisée sur la phase solide est donc en pratique impossible.

Le choix et la préparation des réactifs utilisés, et plus généralement le choix de la phase solide et le procédé d'activation qui lui est appliqué, sont primordiaux pour la mise au point d'un système de détection utilisant une phase solide, qui soit spécifique et sensible, et réellement utilisable et fiable dans la pratique courante des analyses de laboratoire.

La présente invention a pour objet un tel procédé.

La présente invention a pour objet un procédé mettant en oeuvre une mesure indirecte qui consiste à déterminer la consomption induite en milieu liquide par la substance à déterminer, d'un partenaire de liaison primaire présent initialement dans le milieu réactionnel en quantité connue et limitée, et subsistant en partie libre en phase aqueuse après achèvement de la réaction de liaison spécifique avec la substance à déterminer, procédé dans lequel ledit partenaire de liaison subsistant est adsorbé spécifiquement sur une phase solide consistant en un gel de polyacrylamide activé, composé choisi dans le groupe consistant en dialdéhydes, trioxane, paraldéhyde, ledit gel étant également sensibilisé par une substance possédant des propriétés liantes spécifiques pour ledit partenaire primaire. La sensibilisation du gel peut être effectuée indifféremment par un anticorps contre le partenaire de liaison primaire ou encore par une substance identique ou de même nature que la substance à déterminer, ou encore par une substance possédant pour le partenaire de liaison primaire des affinités de liaison spécifiques.

L'essentiel est que le reliquat de partenaire de liaison subsistant après la première réaction d'affinité spécifique, soit adsorbé à son tour spécifiquement sur une phase solide sensibilisée, et que la totalité de cette substance présente sous sa forme libre, et seulement cette forme soit adsorbée.

Il convient donc que la phase solide sensibilisée soit administrée en excès et que cette phase solide soit essentiellement physico-chimiquement inerte.

Selon une caractéristique de l'invention, on utilise un gel de polyacrylamide sensibilisé après activation par un dialdéhyde ou un trioxane ou la paraldéhyde qui est en grande partie saturé après sensibilisation de façon à lui restituer l'inertie physico-chimique nécessaire pour réduire à une quantité négligeable toute absorption non-spécifique indue.

Dans le procédé conforme à l'invention, la détermination de l'activité enzymatique révélatrice se réalise préférentiellement directement sur le gel isolé et lavé de tous contaminants.

Il va de soi que toutes les étapes qui font intervenir un système hétérogène phase liquide phase solide ont lieu sous agitation constante.

Un procédé de détection conforme à l'invention et

applicable à tous haptènes et antigènes ainsi qu'à leurs anticorps spécifiques, ainsi qu'à toutes substances ayant des partenaires de liaison spécifique, et dont la sensibilité est satisfaisante comporte en général les étapes suivantes :

1. - 0,1 à 1 ml d'un liquide physiologique ou d'un tampon contenant une substance à déterminer est amené à un pH adéquat si nécessaire pour que puisse se réaliser une liaison d'affinité spécifique avec un partenaire de liaison. Ceci est achevé par addition éventuelle d'une petite quantité d'un mélange tampon sec. Le pH étant généralement établi entre 5,0 et 9,0. Ce liquide est mélangé avec une certaine quantité d'un tampon p. ex. 0,3 ml habituellement constitué de 1 % d'albumine bovine, 0,85 % de chlorure de sodium et à pH 8,0 contenant une quantité connue et limitée de son partenaire liant. La liaison d'affinité primaire en milieu liquide a lieu durant le laps de temps nécessaire et suffisant et à la température optimale, pour obtenir l'achèvement de la réaction ; les conditions préférentielles sont de 3 à 5 heures à la température de 37°C. Le partenaire qui intervient dans la réaction primaire est présent en la quantité suffisante pour provoquer ultérieurement dans la mesure enzymatique une réaction colorée maximale en l'absence de toute substance liante spécifique. Une mesure ultérieure de la quantité de ce partenaire de liaison subsistant libre après la réaction liante spécifique primaire permet la détermination très exacte de la quantité de substance à déterminer par l'élaboration d'une courbe de référence.

2. - Après achèvement de cette réaction liante d'affinité spécifique primaire en milieu liquide, le mélange réactionnel est mis en contact avec un gel de polyacrylamide sensibilisé par une substance liante spécifique pour le partenaire de liaison primaire. 1 ml d'une suspension de gel de 5 % (V/V°) est centrifugé dans un godet en plastique d'une contenance de 1,5 ml et on élimine le cas échéant le liquide bactériostatique surnageant qui protégeait le gel sensibilisé. Le sédiment est remis en suspension dans le liquide réactionnel. Celui-ci est porté à 1,4 ml par addition d'un tampon à pH 8,0 contenant 1 % d'albumine et 0,85 % de chlorure de sodium.

La phase solide est ajoutée en excès de manière à fixer de façon spécifique tout partenaire de liaison libre. La détermination du taux de présence de certaines substances, tels les anticorps, spécifiques ou autres, peut débuter également par cette opération de fixation sur une phase solide de gel de polyacrylamine sensibilisé, étant entendu que dans ce cas il n'y a pas de réaction de liaison spécifique nécessaire en phase liquide, la liaison du composé à déterminer pouvant intervenir directement sur la phase solide sensibilisée.

L'achèvement de cette fixation du partenaire de liaison libre sur la phase solide est opéré préférentiellement durant 18 à 24 heures à la température ambiante.

3. - La phase solide est ensuite isolée du milieu réactionnel par exemple par centrifugation à 2000 tours/minute durant 20 secondes et lavée une ou deux fois par 1 ml d'une solution tampon à pH 8,0 pour éliminer tous les contaminants qui seraient présents.

4. - Après nettoyage, la quantité du partenaire liant primaire fixé sur la phase solide d'une manière spécifique est mise en évidence par une seconde réaction de liaison spécifique. On peut à cet effet utiliser toute substance qui possède pour ce partenaire de liaison primaire une affinité de liaison spécifique, et communément on utilise un anticorps. L'anticorps révélateur administré en excès dans un ml d'une solution tampon contenant de l'albumine, du chlorure de sodium et amené à pH 8,0 est marqué par un enzyme peroxidase, phosphatase alkaline, malate dehydrogenase, lysozyme, bêta-galactosidase, catalase etc...) ou par un radio-élément tel l'$H^3$, I 125, I 128, soit encore par un fluorophore tel le chlorure de dansyle, le N- (7 dimethylamino-4-methyl-2-oxo-3-chromenyl) maléimide (DACM), l'isothiocyanate de fluoresceine et la dichlorotriazinyl)- aminofluoresceine (DTAF).

5. Après achèvement de cette réaction de liaison spécifique secondaire qui a lieu préférentiellement durant 6 à 7 heures à température ambiante, la phase est isolée du milieu réactionnel par exemple par courte centrifugation et la phase solide est nettoyée deux fois par un ml d'une solution tampon à pH 8,0. La quantité de marqueur présente dans la phase solide est ensuite évaluée par passage de la phase solide dans un compteur de radioactivité, par mesure de la fluorescence ou par une réaction enzymatique selon des méthodes connues en elles-mêmes.

On utilise à cet effet pour obtenir une sensibilité maximale des substances purifiées par immuno-absorption, selon des procédés connus. Une fois purifiée par chromatographie d'affinité, la substance est couplée à un enzyme. Un système de coulage adéquat consiste en une peroxydation contrôlée des carbohydrates présents dans l'enzyme ou le partenaire de liaison secondaire, avec formation subséquente d'une base de Schiff entre les aldehydes créés et les radicaux $-NH_2$ des protéines à conjuguer. La base de Schiff créée est ensuite renforcée par traitement avec un agent réducteur tel le borogydrure de potassium ou le cyanoborohydrure de sodium.

Le second réactif, dont l'inertie physico-chimique doit être maximale est la phase solide activée et sensibilisée. Des essais ont montré que la seule matrice dont l'inertie originelle soit satisfaisante est le gel de polyacrylamide. Un procédé d'activation du gel conforme à l'invention qui respecte en grande partie l'inertie physico-chimique originelle consiste en un traitement du gel par la paraldéhyde, le trioxane ou un dialdéhyde tel que le glutaraldéhyde. L'activation a lieu durant 18 heures à une température comprise entre 37°C et 90°C, préférablement à 60°C, dans une solution de sels minéraux 0,05 à 1 Molaire, préférablement 0,1 M, à pH compris entre 2 et 10 préférablement à pH 6,8. La concentration de l'aldéhyde est de 0,1 % à 10 %, préférablement 5 %, et celle du gel de polyacrylamide est comprise entre 1 % et saturation, préférablement 10 % (V/V, le gel étant humide). Après l'activation, le gel est décanté de façon répétée dans de l'eau distillée pour éliminer les fines particules puis lavé une fois dans un tampon de sels minéraux compris entre 0,05 et 2 Molaire préférablement 0.1M5 à pH compris entre 6 et 10, préférablement à pH 7,5. Le gel est filtré sur du verre fritté. Le gateau humide est ensuite additionné d'une quantité de tampon à pH 7,5 contenant la substance sensibilisante, qui est nécessaire et suffisante pour, après humidification du gel, le couvrir d'un fin film de liquide. La concentration en substance sensibilisante est variable. Typiquement, 50 grammes de gel sec correspondent à 140 grammes de gel au stade de gateau humide, et la totalité de cette matière est sensibilisée par 10 milligrammes d'HCG hautement purifié. La sensibilisation a lieu durant 24 heures à 37°C.

Après sensibilisation, le gel est nettoyé de façon répétée dans de l'eau distillée et dans des tampons à différentes concentrations ioniques pouvant atteindre 2M NaCl et différents pH variant de 4,5 à 8,0. L'activation de la polyacrylamide introduit dans la matrice des groupements chargés qui réduisent l'inertie physico-chimique du gel.

Cette inertie n'est pas restaurée par l'étape de la sensibilisation. Une caractéristique essentielle de l'invention consiste à restaurer l'inertie du gel, après sensibilisation par une protéine ou un haptène, au moyen d'agents chimiques porteurs de groupements -NH$_2$. Les agents chimiques effectifs dans ce but sont l'hydroxylamine, l'acrylamide, la glutamine, l'acetamide et la formamide. La saturation des fonctions aldéhydes libres provenant de l'activation s'effectue en traitant le gel à 10 % (V/V) dans une solution de sels minéraux 0,1 M à pH de préférence 8,0 par une concentration en composés -NH$_2$ variant de 0,0001 à 1 M, préférablement 0,0001 M. Cette réaction dure en général 24 heures à 48 heures à la température ambiante ou à 37°C.

Après saturation par un composé -NH$_2$, le gel est lavé de façon répétée par de l'eau distillée et des tampons, et finalement suspendu à 10 % dans un liquide conservateur.

EXEMPLE 1
Détermination d'HCG

1ml de solutions tampons à pH 8,0 contenant 1 % d'albumine et 0,85 % de chlorure de sodium et ayant une teneur connue en HCG furent incubés durant 3 heures à 37°C avec 350 ng d'anticorps de lapin anti-HCG contenus dans 0,2 ml de tampon à pH 8,0. Le mélange réactionnel fut ensuite incubé durant 18 heures à température ambiante en présence d'une suspension de gel de polyacrylamide sensibilisé par de l'HCG et saturé par de l'acrylamide et maintenu sous agitation constante. Le gel fut ensuite isolé du milieu réactionnel par centrifugation et lavé avec un ml d'une solution tampon à pH 8,0. Après lavage, le gel fut incubé sous agitation constante dans 1 ml d'une solution 1 : 2,000 d'antiglobulines de mouton anti IgG de lapin, marqués à la peroxidase. Après incubation durant 7 heures à la température ambiante, le gel fut isolé par centrifugation et lavé deux fois avec un ml de tampon à pH 8,0. La présence de l'enzyme fixé sur le gel fut révélée par incubation du gel sous agitation constante dans 1,4 ml d'une solution d'O-phenylène diamine à 0,1 % dans un tampon citrate 0,2 M à pH 5,0 contenant 1 ml d'$H_2O_2$ à 30 volumes.

Après 50 minutes à température ambiante, la réaction enzymatique fut stoppée par addition de 100 µl de $H_2SO_4$ à 50 % et la phase solide éliminée par centrifugation. Le produit de réaction enzymatique fut évalué par lecture de la densité optique à 480 nm.

Le résultat de cette analyse montre que l'HCG peut être déterminé par cette méthode entre 1 mUI ml et 256 mUI/ml.


EXEMPLE II

Détermination de LH équin par une réaction immunologique hétérologue

0,1 ml d'une solution contenant 50 µg/ml d'anticorps de lapin anti-LH de mouton fut mis en contact avec 1 ml d'une suspension à 3 % d'un gel de polyacrylamide dans du tampon à pH 8,0 contenant 1 % d'albumine, sensibilisé au LH de jument et saturé par de la glutamine. Après incubation sous agitation constante à température ambiante durant 18 heures, le gel fut isolé par centrifugation, lavé une fois comme décrit dans l'exemple I et incubé durant 7 heures à température ambiante avec une solution 1 : 2,000 d'antiglobulines de mouton anti IgG de lapin marqués à la peroxidase. Après incubation, le gel fut isolé et lavé comme décrit précédemment, et la présence d'une activité enzymatique fixée sur la phase solide fut détectée, comme décrit précédemment. La densité optique mesurée fut de 0,7. Une incubation préalable en milieu liquide durant 3 heures à 37°C d'une mélange réactionnel consistant en 0,1 ml d'anticorps de lapin anti-LH de mouton (50 µg/ml) avec 0,1 ml d'une solution à 20 mUl/ml de LH equin, suivi de l'absorption des anticorps libres subsistants, sur une phase solide tel que décrit ci-dessus, résultat en une chute de la densité optique de 15 %. Une telle chute est considérée comme la limite de sensibilité du système.

EXEMPLE III

Détermination d'oestradiol

De l'oestradiol-6- (-O-carboxymethyloxyme) conjugué à de l'oval bumine fut préparé selon une méthode connue (Erlanger, B.F. et al., in : Methods in Immunology and Immunochemistry, Williams and Chase, Ed. Vol I, pp. 144-151 Academic Press N.Y.), et le conjugué fut utilisé pour sensibiliser un gel de polyacrylamide, saturé ensuite par de la glutamine.

Des anticorps de lapin anti-oestradiol sont obtenables dans le commerce (Sigma, St. Louis, Missouri 63178 U.S.A.). Après réaction en milieu liquide durant 3 heures à 37°C entre des doses connues d'oestradiol et de l'anticorps solubilisés dans 1 ml de tampon-Albumine à pH 8,0, l'anticorps libre subsistant fut adsorbé sur le gel sensibilisé comme décrit dans les exemples précédents. La révélation de l'activité peroxidasique localisée dans la phase solide fut réalisée par incubation du gel sous agitation constante dans 1 ml d'acide homovallinique à 0,25 % dans un tampon Tris-HCl 0,1 M à pH 8,0 additionné de 0,1 ml d'une solution 0,05 % d'$H_2O_2$. Après incubation durant 60 minutes à température ambiante, le gel solide fut éliminé par centrifugation et l'intensité de la fluorescence présente dans le surnageant fut mesurée à 320 nm (excitation) et 420 nm (émission). Les résultats de cette analyse montrèrent que l'oestradiol est déterminable par la méthode décrite, entre 50 pg/tube et 1 ng/tube.

EXEMPLE IV

Détermination qualitative et quantitative d'antiglobulines humaines

Un gel de polyacrylamide activé par la paraldéhyde est sensibilisé par des gamma-globulines equines, et ensuite saturé par de l'acrylamide, en accord avec l'invention.

Un ml d'une suspension de gel à 5 % (V/V) est centrifugé et le sédiment est incubé en présence de 0,15 ml de sérum humain provenant d'une personne atteinte d'arthrite rhumatoïde, dilués à 1 ml par un tampon-Albumine pH 8,0 comme décrit dans les exemples précédents. Après incubation durant 3,5 heures à 37°C, le gel est isolé par centrifugation et lavé, comme décrit dans les exemples précédents. La nature des anti-globulines humaines fixées sur le gel par une liaison d'affinité spécifique avec les gamma-globulines équines fut établie par une incubation de la phase solide dans 1 ml d'une solution d'anticorps IgG de lapin anti-IgG humain ou anti IgM humain à dilution appropriée. Il est essentiel que l'anticorps de lapin utilisé soit purifié et rendu absolument spécifique par une immuno-adsorption sur gamma-globulines humaines insolubles IgM ou IgG. Ces anticorps peuvent être obtenus dans le commerce (Institut Pasteur Production) et purifiés selon des méthodes bien établies.

Après incubation durant six heures à température ambiante en présence de ces anticorps de lapin spécifiques pour des gamma-globulines humaines IgM ou IgG, le gel est isolé du milieu réactionnel, lavé comme décrit précédemment et incubé en présence d'anticorps de mouton anti-IgG de lapin, marqués à la peroxidase, comme décrit dans les exemples précédents, et la présence d'antiglobulines humaines spécifiques présentes sur le gel est révélée par une intensité de coloration accrue.

La quantification du procédé exige l'élaboration de deux courbes de référence qui déterminent avec exactitude la quantité de gamma-globulines de lapin anti-IgG humain ou anti-IgM humain fixé sur les gels par une liaison spécifique puisque cette quantité reflète la quantité d'antiglobulines humaines liées aux globulines équines et détermine la quantité du complexe anticorps de mouton-peroxidase qui se fixera sur le gel dans une réaction liante d'affinité spécifique pour les anticorps de lapin.

Pour l'élaboration de ces courbes de référence, on sensibilise un gel de polyacrylamide par des gamma globulines humaines de type G ou M. Des quantités connues présentes en concentrations décroissantes de gamma-globulines de lapin monospécifiques, purifiées par chromatographie d'affinité, sont mises en contact avec ces gels sensibilisés et fournis en excès dans

0011030

un protocole d'expérience identique à celui où le gel sensibilisé aux gamma-globulines équines est utilisé. La révélation de la présence des gamma-globulines de lapin par des anticorps de mouton anti IgG de lapin marqués à la péroxydase a lieu de la même manière, et ceci permet l'élaboration des deux courbes de références nécessaires pour une détermination quantitative.

L'application de la méthode utilisée selon l'invention a permis la détection d'une quantité d'antiglobulines spécifiques de la classe IgG égale à 10 ng/ml de sérum, et de la classe IgM égale à 2 ng par ml de serum analysé.

REVENDICATIONS.

1.          Procédé pour la détermination de substances
montrant entr'elles des affinités de liaison spécifiques,
dans lequel on soumet à une réaction de liaison spécifique la
substance à déterminer avec un partenaire de liaison primaire
en quantité connue et limitée, une partie dudit partenaire de
liaison primaire subsistant après achèvement de la réaction
de liaison spécifique, caractérisé en ce que ledit partenaire
de liaison subsistant est adsorbé spécifiquement sur une phase
solide consistant en un gel de polyacrylamide sensibilisé par
une substance ayant des propriétés liantes spécifiques pour le
partenaire de liaison primaire puis détecté par voie enzymatique ou par fluorescence.

2.          Procédé selon la revendication 1, caractérisé
en ce que le gel de polyacrylamide est activé par un composé
choisi dans le groupe consistant en dialdéhydes, trioxane,
paraldéhyde.

3.          Procédé selon l'une quelconque des revendications 1 et 2, caractérisé en ce que le gel de polyacrylamide
est saturé après sensibilisation.

4.          Procédé selon la revendication 3, caractérisé
en ce que le gel est saturé par un composé porteur de groupements -$NH_2$ choisi dans le groupe consistant en hydroxylamine,
acrylamide, glutamine, acétamide et formamide.

5.          Procédé selon l'une quelconque des revendications 3 et 4, caractérisé en ce que la saturation du gel est
opérée dans une solution de sels minéraux à 0,1 M à pH 8, avec
une concentration en composé saturant de 0,0001 à 1 M, pendant
une durée comprise entre 24 et 48 heures et une température
comprise entre 20 et 37°C.

6.          Procédé selon l'une quelconque des revendications 1 à 5, caractérisé en ce que la détection par voie enzymatique ou par fluorescence comprend l'étape consistant à coupler un fluorophore ou un enzyme avec un partenaire de liaison
spécifique secondaire pour le partenaire de liaison primaire,
ledit partenaire de liaison secondaire étant purifié par chromatographie d'affinité, ledit couplage étant effectué par peroxydation des carbohydrates présents dans l'enzyme ou dans le
partenaire de liaison spécifique secondaire puis adjonction

- 2 -

REVENDICATIONS

d'un composé choisi parmi le borohydrure de potassium et le cyanoborohydrure de sodium.

0011030

Numéro de la demande

EP 79 40 0796

## RAPPORT DE RECHERCHE EUROPEENNE

Office européen
des brevets

| Catégorie | Citation du document avec indication, en cas de besoin, des parties pertinentes | Revendication concernée |
|---|---|---|
| | **DOCUMENTS CONSIDERES COMME PERTINENTS** | |
| | FR - A - 2 228 221 (WISCONSIN ALUMNI RESEARCH FOUNDATION) <br> * Revendications 1,3,6 * | 1 |
| | -- | |
| | US - A - 3 951 748 (R.F. DEVLIN) <br> * Colonne 15, revendications 1,3,4 * | 1,2,4 |
| | -- | |
| A | US - A - 4 016 043 (A.H.W.M. SCHUURS et al.) <br> * Brevet en entier * | 6 |
| | -- | |
| A | US - A - 3 793 445 (S.J. UPDIKE) <br> * Colonnes 1-2 * | 1 |
| | ---- | |

**CLASSEMENT DE LA DEMANDE (Int. Cl)**

G 01 N 33/54

**DOMAINES TECHNIQUES RECHERCHES (Int. Cl)**

G 01 N 33/54
G 01 N 33/74
G 01 N 33/76
G 01 N 33/78

**CATEGORIE DES DOCUMENTS CITES**

X particulièrement pertinent
A arrière-plan technologique
O divulgation non-écrite
P document intercalaire
T théorie ou principe à la base de l'invention
E demande faisant interférence
D document cité dans la demande
L document cité pour d'autres raisons
& membre de la même famille, document correspondant

Le présent rapport de recherche a été établi pour toutes les revendications

| Lieu de la recherche | Date d'achèvement de la recherche | Examinateur |
|---|---|---|
| La Haye | 29.01.1980 | MEYLAERTS |

OEB Form 1503.1 06.78